(19) Europäisches Patentamt
European Patent Office
Office européen des brevets

(11) Publication number: **0 582 458 B1**

(12) **EUROPEAN PATENT SPECIFICATION**

(45) Date of publication of patent specification: **22.11.95**

(51) Int. Cl.$^{6}$: **A61K 31/585**, A61K 47/18, A61K 7/06

(21) Application number: **93306120.2**

(22) Date of filing: **03.08.93**

(54) **Compositions for the topical administration of spironolactone.**

(30) Priority: **06.08.92 US 926561**

(43) Date of publication of application:
**09.02.94 Bulletin 94/06**

(45) Publication of the grant of the patent:
**22.11.95 Bulletin 95/47**

(84) Designated Contracting States:
**AT BE CH DE DK ES FR GB GR IE IT LI LU NL PT SE**

(56) References cited:
**EP-A- 0 028 525**
**EP-A- 0 124 147**
**EP-A- 0 273 202**
**EP-A- 0 410 348**
**WO-A-92/07589**
**FR-A- 2 588 755**
**US-A- 4 347 245**

(73) Proprietor: **Beta Pharmaceuticals Co.**
**Juncal 1305**
**1201 Montevideo (UY)**

(72) Inventor: **Carrara, Dario Norberto Ramon , Dr.**
**Verdi 2175**
**Hurlingham,**
**Prov. of Buenos Aires (AR)**
Inventor: **Stefano, Francisco Jose Evaristo, Dr.**
**Las Heras 3807**
**Buenos Aires (AR)**

(74) Representative: **Pearce, Anthony Richmond**
**MARKS & CLERK,**
**Alpha Tower,**
**Suffolk Street Oueensway**
**Birmingham B1 1TT (GB)**

EP 0 582 458 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

This invention relates to the administration of a systemically active antiandrogen, namely spironolactone, through the skin. The invention reveals a pharmaceutical formulation with good cosmetic properties and low irritation potential, useful for the tropical treatment of acne vulgaris, seborrheic dermatitis and hirsutism. A preparation that delivers spironolactone, at a rate that would ensure therapeutic concentrations, at the site of action (the sebaceous glands), but will not produce an important spillover to the general circulation (to avoid undesirable systemic effects), should contain defined amounts of chemicals that minimize the barrier characteristics of the uppermost layer of the epidermis. Said chemicals are: oleic acid, 1-methyl-2 pyrrolidone, 2,2-dimethyl octanoic acid and N,N dimethyl lauramide/propylene glycol monolaureate or combinations thereof. As utilized throughout the description, the term "spironolactone" refers to aldactone, 3-(3-oxo-7α-acetylthio-17β-hydroxy-androst-4-en-17α-yl ) propiolactone having the formula:

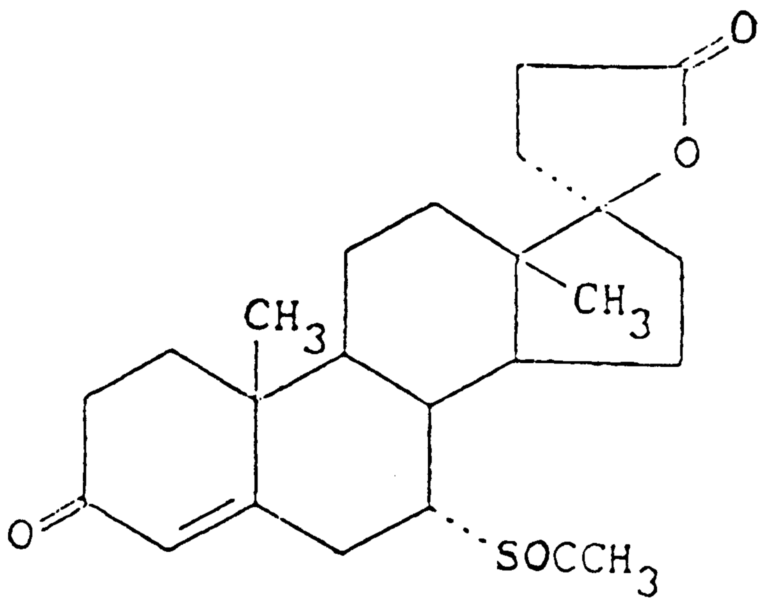

### BACKGROUND OF THE INVENTION

The present invention relates to the topical treatment of acne vulgaris, seborrhea and hirsutism with steroid spironolactone and, in particular, to ways of providing therapeutically useful concentrations of the antiandrogen at its site of action, the sebaceous gland, whilst maintaining the spillover of the drug into the blood stream at a very low level, thus preventing systemic side effects.

Seborrheic acne is a dermatological disease with high prevalence in teenagers of both sexes. It is characterized by the presence of inflammatory lesions usually localized in cheeks and forehead. These lesions are often accompanied by comedones. When bacterial growth is prominent, the lesions become suppurative. In the most severe cases, the lesions are spread over the back and chest. In these forms of acne there are formations of cysts with purulent accumulations that connect each other subcutaneously.

Due to its potential for leaving scars together with the profound effect that this has on teenager personality, it is important to treat acne and seborrheic condition.

High doses of oral spironolactone have been successfully used in the treatment of acne as well as hirsutism (Shaw, 1991).

The bibliographical information related to the treatment of these affections by means of antiandrogens reveals that until now, said treatment has been confined to:

(i) the administration of spironolactone "per os" that reaches the hypodermis, the dermis and the most deep layers of epidermis to compete with or inhibit the tissular testosterone; and

(ii) topical applications of formulations containing spironolactone. The previously mentioned limited permeation of the epidermis external layer explains the reduced penetration of the spironolactone and

the poor effects of said topical applications. The systemic use of spironolactone, however can cause a variety of side effects, endocrine (since it is an aldosterone antagonist), neurologic and gastrointestinal, with an incidence that ranges from 75 to 91 % (Muhlemann, 1986; Hughes, 1988).

To circumvent these effects topical spironolactone has been tried (Messina, 1983; Califano, 1990; Pizzino, 1987).

However, this administration route proved not to be successful in view of the scarcity of publications and lack of commercial success. Also Walton (Walton, 1986) proved that commercially available preparations did not reduce sebum excretion. The hypothesis can be raised that these failures are related to the fact that the formulations used did not provide suitable permeation of the applied steroid to the site of action and that most of the spironolactone remained in the outer surface of the skin without crossing the barrier imposed by the stratum corneum. It should be noted that the skin separates the internal organs from the outside environment and serves as a protective barrier against the penetration of chemicals. It is an organ composed of many histological layers, and is described in terms of 3 mayor multilaminate layers: the epidermis, the dermis and the hypodermis. The epidermis is composed of five strata: stratum germinativum, stratum spinosum, stratum granulosum, stratum lucidum and stratum corneum. The later consists of many layers of compacted, flattened, dehydrated and keratinized cells that are rather physicologically inactive and continuously shed and replenished from the innermost layers.

These layers, due to the physicochemical properties of their cells are the main resistance to the entrance of external agents and act as an efficient barrier. Molecules moving from the environment into the skin must first penetrate the stratum corneum and migrate following a concentration gradient. To increase skin permeability and in particular the permeability of the stratum corneum, and the amount of drug delivery by topical applications, the skin may be treated either with the applications of one or more permeation enhancer agent or else the drug and the permeation enhancer may be applied together.

Another approach for increasing the amount of drug delivered into the skin might be to include a higher concentration of the pharmaceutically active drug in the pharmaceutical preparation.

The increase in the concentration of the drug would hopefully augment the amount permeated. This concept might work up to a certain extent but is limited by the amount of drug which can be permeated through the skin barrier, that acts as a rate limiting step.

Various compounds for enhancing the permeability of skin are known in the art.

Perhaps the most famous of such penetration enhancer is DMSO (dimethyl sulfoxide). However DMSO has not received FDA approval. Another well known substance is Azone(R), see US Patent Nos. 3909816,1-dodecylhexahydro-2H-azepin-2-one (Nelson Research Development); 4311481 and 4316893. US Patent N. 4537776. Cooper, 1985, has an excellent summary of prior art in the use of binary systems for permeability increase of the stratum corneum.

Recently Cooper, in "Increased skin permeability for lipophilic molecules", published in Journal of Pharmaceutical Sciences Vol. 73 No. 88 (1984), discloses the use of oleic acid as a penetration enhancer in the presence of propylene glycol (see also US Patent No. 4537776).

Neodecanoic acid has been shown to be a potent enhancer of naloxone permeation with a low irritation profile (Aungst, 1989). Also pyrrolidone derivatives have been shown to increase the permeability of the skin to several compounds by Sasaki, 1990, and also lauramide derivatives by Mirejovsky and Takruri, 1986.

However none of the above mentioned inventions or publications deals with the study of spironolactone.

Moreover as pointed by Chien in "Transdermal controlled Systemic Medications", Marcel Dekker INC, New York 1987, pages 69-75, an enhancer increases the permeation of different compounds to different degree. There are not such general or universal enhancers. As an example we can quote results of this author as wherein below indicated:

| COMPOUND | ENHANCEMENT FACTOR BY AZONE | ENHANCEMENT FACTOR BY PROPYLOLEATE | ENHANCEMENT FACTOR BY PROPYL MYRISTATE |
|---|---|---|---|
| Progesterone | 6.0 | 5.4 | 4.6 |
| Estradiol | 20.2 | 14.6 | 9.3 |
| Hydrocortisone | 61.3 | 5.0 | 4.6 |
| Indomethacin | 14.5 | 4.7 | 3.8 |

This type of result clearly indicates that optimal permeation of a given compound can be achieved only by careful experimentation as shown in our Argentinian Patent Applications N. 315.433 and 314.479

DETAILED DESCRIPTION

The present invention relates to a spironolactone-containing composition for application to an area of human skin afflicted with acne, seborrheic condition or hirsutism for effective treatment thereof.

Applicants have discovered that the addition of N,N-dialkyllauramides, particularly the N,N-dimethyl lauramide as skin permeation enhancers to said composition greatly increases the drug availability in relation to its site of action and that, at the same time, a residual action is obtained. The topical preparation herein described will have therapeutic effects but will avoid the undesirable side effects of the drug when administered orally.

The present invention proposes a medicament for the treatment of testosterone-mediated skin disorders, administrering in a sustained and controlled manner spironolactone directly to the dermal tissues where the testosterone is found. In this way, it is possible to have effective amounts of antiandrogens in the required areas, avoiding the inconvenience of systemic route and the slowness of topical applications without employing the enhancer. The composition according to this invention comprises effective amounts, for example, 0.1 to 10% w/w, of spironolactone and N,N-($C_1$-$C_4$) dialkyllauramide as skin permeation enhancer for spironolactone, in a mixture with (1) emulsifying agents (2) solvent for spironolactone (3) buffer salts in amounts necessary to maintain a pH between 4.0 and 6.0 (4) water (5) preservative agents.

In order for a compound to be useful as a skin permeation enhancer the compound must meet a number of different requirements. Firstly, the compound must be a dermatologically acceptable, so, when used topically on the skin, , not to cause any adverse side effect to it. Secondly, the compound must be compatible with spironolactone present in the pharmaceutical. Thirdly, the compound must have a substantial effect on increasing the permeability of spironolactone.

According to the present invention, the use of N,N-dimethyl lauramide has been studied as enhancer of the residual effect of permeation of spironolactone. It has been observed that N,N-di (C1-C4) alkyl lauramide, and particularly N,N-dimethyl lauramide, works as an enhancer of permeation with an effectively higher activity than that of other enhancer compounds.

Additionally, it has a residual effect on spironolactone, which has not been know so far. The N,N-dimethyl lauramide substantially differs from other known permeation enhancers in that it noticeably increases the permeation of spironolactone as compared with other known permeation enhancers. In addition, it involves a residual action of the active drug, spironolactone, which is wholly novel regarding topical application of said drug. The term "residual action" refers to the persistence of the effect of the active drug, such as spironolactone, in almost equal intensity in spite of the removal of the active drug.

It has been researched and finally determined that the action of N,N-dimethyl lauramide with spironolactone can be further improved if used in combination with a specific additional enhancer.

The additional enhancer preferably is one of the known permeation enhancers described above and preferably is a fatty acid having 14 to 22 carbon atoms and one or more double bonds, a di (C1-C4) alkyl substituted fatty acid having 8 to 14 carbon atoms, N-methyl pyrrolidone, or combinations of any of the foregoing. Most preferred additional enhancers are oleic acid, N-methyl pyrrolidone and neodecanoic acid.

Preferably, the amount of spironolactone will range from 0.1 to 10 percent w/w. Preferably, the amount of N,N-dimethyllauramide will be in the range of o.1 to 10% w/w. Preferably, the amount of additional enhancer will range from 0.1 to 10 percent w/w and most preferably from 2.5 to 10 percent w/w.

Also contemplated are pharmaceutical compositions comprising a safe, effective amount of spironolactone, preferably 0.1 to 10 percent w/w; a permeation enhancer comprising: N,N-di (lower or C1-C4 alkyl) lauramide and a pharmaceutically acceptable carrier; a pharmaceutical composition as above mentioned which can optionally comprise an additional enhancer. The pharmaceutically acceptable carrier includes one or more of buffers, preservatives, flavoring additives, and excipients in order to provide a cream having a cosmetic appearance.

The following formulation illustrates a composition according to the present invention.

The cream or ointment to which both spironolactone and N,N-dimethyl lauramide, the skin permeation enhancer are added for the purposes of this invention has the following general composition.

| | |
|---|---|
| Emulsifying wax | 8 - 20 wt. percent |
| Liquid petrolatum | 2 - 10 wt. percent |
| White petrolatum | 4 -12 wt. percent |
| Propyl Paraben | 0.05 - 0.1 wt. percent |
| Methyl Paraben | 0.15 - 0.20 wt. percent |
| Dihydrate sodium citrate | 0.6 -1 wt. percent |
| Citric acid, mohydrate | 0.2 - 0.7 wt. percent |
| Purified water | 55 - 75 wt. percent |
| Spironolactone | 05 - 10 wt. percent |
| Permeation enhancer - N,N-dimethyl lauramide | 1 - 12 wt. percent |

If necessary, up to 10% w/w of oleic acid or N-mehtyl pyrrolidone as additinal enhancer, can be added to the formulation indicated in the above Table.

Methods for Determining the Permeation Ratio of Spironolactone

Male mice, 8 to 16 weeks of age were shaved on their abdominal skin 72 hrs. before sacrifice by cervical dislocation. Only animals that showed absence of irritative lesions produced by the shaving were used. A section of full thickness abdominal skin was surgically excised and mounted between the two sections of a vertical diffusion cell having 2.2 cm2 surface area, the epidermal layer facing up. A given amount of the pharmaceutical preparation containing different concentrations of spironolactone and a selected enhancer was spread over the epidermal layer whilst the dermal layer was in contact with a solution of ethanol/phosphate buffer pH 7.4 40% v/v, at 34ºC. The presence of spironolactone in the inferior cup (receptor phase) was monitored taking samples at given times and measured afterwards with an HPLC method.

Example 1

A solution of 1 mg/ml or ≈ 0.1% spironolactone in propylenglycol was put in the upper cell of the diffusion apparatus and the flux of spironolactone measured during the following 48 hrs. The flux was very low and not compatible with a therapeutic effect on the sebaceous glands. However, if oleic at a concentration of 10% is added to this solution there is a marked enhancement of the flux of spironolactone through the stratum corneum.

TABLE 1

PERMEATION OF SPIRONOLACTONE THROUGH MOUSE SKIN FROM A 1 mg/ml SOLUTION IN PROPYLENGLYCOL expressed as µg/cm2

| TIME hrs | PROPYLENGLYCOL | PROPYLENGLYCOL + OLEIC ACID |
|---|---|---|
| 6 | 1.3 ± 0.2 | 87.0 ± 10.3 |
| 24 | 26.1 ± 16.8 | 523.0 ± 10.0 |
| 48 | 43.5 ± 14.3 | 1096.0 ± 200.0 |

This example shows that spironolactone has a very low permeability, that prevents the drug to reach its site of action, although this inconvenient can be overcome with the addition of an enhancer.

Example 2

A cream containing 5% spironolactone was charged on top of the diffusion apparatus. The composition of the cream was as follows: Spironolactone 5%, mixture of cetearylalcohol and sodium laurylsulphate 9%, light mineral oil 6%, diisopropyl adipate 4.5%, white petrolatum 10.5%, mixture of glycerolmonoestearate and polyoxyethylene stearate 5%, propylparaben 0.05%, methylparaben 0.15%, citric acid monohydrate 0.5%, sodium citrate dehydrate 0.65% and water to 100%.

The cream thus prepared had excellent cosmetic appearance.

TABLE 2

PERMEATION OF SPIRONOLACTONE THROUGH MOUSE SKIN FROM A PHARMACEUTICAL COMPOSITION CONTAINING 5% SPIRONOLACTONE expressed as µg/cm2

| TIME hrs | SPIRONOLACTONE PERMEATED |
|---|---|
| 3 | 5.6 ± 1.8 |
| 8 | 33.0 ± 5.5 |
| 24 | 354.0 ± 20.0 |

As shown in Table 2 spironolactone released from this pharmaceutical preparation crossed at a relative slow rate the skin.

Example 3

In this example, the effect of adding either 2.5% or 5% or 10% isopropyl myristate to the pharmaceutical preparation above described was analyzed.

TABLE 3

SPIRONOLACTONE Permeation expressed as µg/cm2

| TIME hrs | ISOPROPYL MYRISTATE CONTENT | | | | | | | |
|---|---|---|---|---|---|---|---|---|
| | (n) | NONE | (n) | 2.5% | (n) | 5% | (n) | 10% |
| 3 | 22 | 9.0 ± 1.6 | 5 | 13.2 ± 6.5 | 8 | 9.5 ± 176.0 | 10 | 17.3 ± 2.5 |
| 8 | 22 | 64.7 ± 7.5 | 5 | 59.6 ± 17.6 | 8 | 56.1 ± 10.4 | 10 | 90.3 ± 12.4 |
| 24 | 22 | 435.0 ± 23.0 | 5 | 419.0 ± 44.0 | 8 | 516.0 ± 98.0 | 10 | 811.0 ± 163.0 |

Values are MEAN ± SEM, n indicated number of experiments.

As shown in Table 3, only the highest concentration of isopropyl myristate produced an important increase in the flux of spironolactone across mouse skin.

Example 4

In this example the effect of adding either 2.5% or 10% of propylenglycol to the pharmaceutical preparation above described, was analyzed.

Propylenglycol added to the pharmaceutical composition of example Nº 1 in the concentrations range between 2.5% and 10% did not produce an increased flux of spironolactone (see Table 4).

TABLE 4

SPIRONOLACTONE PERMEATION expressed as µg/cm2

| TIME | PROPYLENE GLYCOL CONTENT | | | | | | | |
|---|---|---|---|---|---|---|---|---|
| | (n) | NONE | (n) | 2.5% | (n) | 5% | (n) | 10% |
| 3 | 22 | 9 ± 1.6 | 5 | 8.4 ± 0.5 | 6 | 4.5 ± 0.6 | 4 | 4.2 ± 0.6 |
| 8 | 22 | 64.7 ± 7.5 | 5 | 60.0 ± 11.0 | 6 | 26.5 ± 4.5 | 4 | 29.0 ± 4.7 |
| 24 | 22 | 434.8 ± 23.3 | 5 | 376.0 ± 47.0 | 6 | 422.0 ± 41.0 | 4 | 387.0 ± 93.0 |

Example 5

The effects of several concentrations of oleic acid on spironolactone permeation were tested in conditions similar to those of the precedent example.

TABLE 5

SPIRONOLACTONE PERMEATION expressed as µg/cm2

| TIME | OLEIC ACID CONCENTRATION | | | | | | | |
|---|---|---|---|---|---|---|---|---|
| hrs | (n) | NONE | (n) | 2.5% | (n) | 5% | (n) | 10% |
| 3 | 22 | 9 ± 1.6 | 5 | 8.7 ± 0.6 | 8 | 11.0 ± 1.0 | 9 | 22.0 ± 4.7 |
| 8 | 22 | 64.7 ± 7.5 | 5 | 67.0 ± 5.6 | 8 | 62.0 ± 8.4 | 9 | 123.0 ± 25.4 |
| 24 | 22 | 434.8 ± 23.3 | 5 | 523.0 ± 44.0 | 8 | 764.0 ± 99.0 | 9 | 1101.0 ± 151.8 |

In contrast to the results obtained with isopropyl myristate or propylenglycol, where either a slight increase or no increase in permeation was observed, oleic acid at 5% and 10% concentration produced a marked increase in permeation that was evident already at 3 hours.

Example 6

Again, and following the procedure of the preceding examples a series of tests utilizing either neodecanoic acid or N-methyl pyrrolidone were performed. The results showed that both compounds were less potent than oleic acid to increase permeation at early times although proved similar efficacy at 24 hrs.

TABLE 6

SPIRONOLACTONE PERMEATION expressed as µg/cm2

| TIME hrs | N METHYL PYRROLIDONE CONCENTRATION | | | |
|---|---|---|---|---|
| | (n) NONE | (n) 2.5% | (n) 5% | (n) 10% |
| 3 | 22 9.0 ± 1.6 | 5 11.4 ± 2.2 | 3 14.1 ± 1.5 | 4 7.7 ± 1.4 |
| 8 | 22 64.7 ± 7.5 | 5 56.5 ± 14.8 | 3 116.6 ± 12.2 | 4 56.9 ± 8.8 |
| 24 | 22 434.8 ± 23.3 | 5 443.9 ± 55.4 | 3 667.8 ± 70.4 | 4 993.8 ± 243.3 |

TABLE 6 B

SPIRONOLACTONE PERMEATION expressed as µg/cm2

| TIME hrs | NEODECANOIC ACID CONCENTRATION | | |
|---|---|---|---|
| | (n) 2.5% | (n) 5% | (n) 10% |
| 3 | 5 10.9 ± 3.2 | 9 8.0 ± 0.9 | 9 10.2 ± 2.3 |
| 8 | 5 88.0 ± 27.0 | 9 70.5 ± 8.0 | 9 86.8 ± 21.0 |
| 24 | 5 879.0 ± 253.6 | 9 661.0 ± 83.0 | 9 925.7 ± 153.8 |

Example 7

Once more, a similar test was performed using different concentrations of N,N-dimethyl lauramide. At the relative low concentration of 5% this compound showed a clear effect after 8 hrs. of incubation reaching a maximum effect with this concentration (TABLE 7).

| TABLE 7 SPIRONOLACTONE PERMEATION expressed as $\mu g/cm2$ | | | | | | | | |
|---|---|---|---|---|---|---|---|---|
| TIME hrs | DIMETHYL LAURAMIDE CONCENTRATION | | | | | | | |
| | (n) | NONE | (n) | 2.5% | (n) | 5% | (n) | 10% |
| 3 | 22 | 9 ± 1.6 | 5 | 10.0 ± 1.8 | 8 | 12.2 ± 2.8 | 10 | 17.4 ± 3.8 |
| 8 | 22 | 64.7 ± 7.5 | 5 | 75.4 ± 9.0 | 8 | 116.3 ± 21.5 | 10 | 88.8 ± 11.1 |
| 24 | 22 | 434.8 ± 23.3 | 5 | 777.2 ± 202.8 | 8 | 1037.4 ± 162.1 | 10 | 1195.7 ± 141.3 |

Example 8

To test if prior application of the enhancer (N,N-dimethylllauramide) will increase further the permeation of spironolactone the following experiments were performed.

The abdominal skin of mice was shaved and during 72 hrs the area was covered 3 times per day with the pharmaceutical composition described in Example 3 (control group) or with this composition containing N,N-dimethyl lauramide. At the end of the period, the area was cleaned with water, excised and mounted in the cells. In these conditions, we measured the permeation of spironolactone from either a cream containing 5% spironolactone or 5% spironolactone plus 10% N,N-dimethyl lauramide.

As judged by the results reported in Table 8 the pretreatment greatly enhanced the permeation of spironolactone.

| TABLE 8 SPIRONOLACTONE PERMEATION expressed as ratio | |
|---|---|
| TIME hrs | PERMEATION RATIO PRETREATED DML / PRETREATMENT CONTROL |
| 3 | 6.5 ± 3.3 |
| 6 | 4.3 ± 1.8 |
| 24 | 4.1 ± 0.6 |

Example 9

In this example the effect of different concentrations of propylenglycol monolaureate are demonstrated.

TABLE 9

SPIRONOLACTONE PERMEATION expressed as µg/cm2

| TIME hrs | PROPYLENGLYCOL MONOLAUREATE | | | |
|---|---|---|---|---|
| | NONE | 2.5% | 5% | 10% |
| 3 | 3 ± 0.07 | 8 ± 2.7 | 7 ± 1.7 | 17 ± 5 |
| 8 | 34 ± 3.6 | 60 ± 13 | 56 ± 22 | 118 ± 27 |
| 24 | 423 ± 29 | 487 ± 61 | 550 ± 113 | 933 ± 114 |

These results clearly show that this enhancer has a potent effect in the early times. Thus the enhancing factor of 10% was at 3 hours 5.6 and decreased to 2.2 at 24 hours. These results indicate a distinctive effect of this enhancer of great value for a topical cream.

Supplementary tests were made in order to compare the permeation ratio of spironolactone over that of the oleic acid (OA) and that of neodecanoic acid (NDA), two known enhancers used in the prior art, which have already been referred to (vide supra).

Table 10 refers to permeation measured on samples according to the present invention (DML 10% w/w) and the like with 10% oleic acid (OA) and 10 % neodecanoic acid (NDA) under similar conditions. Skin was pre-treated with each enhancer, as described for Table 8.

The values in Table 10 show, apart from an absolute permeation (micrograms/$cm^2$) of permeation DML as compared with OA and NDA, a greater relative permeation (quotient of absolute permeation in Table 10 and control values), as shown on Table 11.

Further, the effects of pre-treatment on the enhancer as anticipated for Table 8, are the confirmed when comparing the absolute permeation found at 2194.4 micrograms/$cm^2$ for DML in Table 10 with permeation found for Table 7 at 1195.7 micrograms/$cm^2$ without pre-treatment.

| TABLE 10 | | | | |
|---|---|---|---|---|
| SPIRONOLACTONE PERMEATION expressed as $\mu g/cm^2$ | | | | |
| WITH PRETREATMENT | | | | |
| TIME hrs | Control | DML 10% | OA 10% | NDA 10% |
| 3 | 12.8 ± 5.6 | 83.8 ± 40.2 | 17.0 ± 2.5 | 6.0 ± 1.8 |
| 8 | 95.4 ± 20.5 | 415.7 ± 163.1 | 142.2 ± 16.7 | 78.1 ± 26.0 |
| 24 | 534.3 ± 96.6 | 2194.4 ± 316.7 | 1353.1 ± 125.5 | 1092.3 ± 143.1 |

| TABLE 11 | | | |
|---|---|---|---|
| SPIRONOLACTONE PERMEATION RATIO/CONTROL | | | |
| WITH PRETREATMENT | | | |
| TIME hrs | DML 10% / Control | OA 10% / Control | NDA 10% / Control |
| 3 | 6.52 | 1.33 | 0.47 |
| 8 | 4.36 | 1.49 | 0.82 |
| 24 | 4.11 | 2.53 | 2.04 |

While the above examples illustrate numerous embodiments of the invention, the scopes are limited only by the operability exhibited by improved permeation of spironolactone attributable to the incorporation of a chemical such as N,N-dimethyllauramide to a common pharmaceutical preparation.

Also the results presented in these application clearly prove that at least for spironolactone there is no such an universal enhancer and that adequate fluxes across the skin can be achieved only by testing different types of compounds. Although prior art was useful for the theoretical approach, the results emerged from the careful investigation of multiple variables.

## REFERENCES


1. Shaw J. C., J. Am. Acad. Dermatol. 1991, 24, 236-243.
2. Muhlemann M. F., Carter G. D., Cream J. J. et al, British J.Dermatology 1986, 115, 227-232.
3. Hughes B. R., Cunliffe W. J., British J. Dermatology 1988, 118, 687-691.

4. Messina M., Manieri C., Rizzi G. and G. M. Molinatti, Current Therapeutic Research 1983, 34, 319-324.
5. Califano L, Cannavo S., Siragusa M, Gerardi R., Clin. Ther. 1990, 135, 193-199.
6. Pizzino D., Bettoli V., Varotti C., Giornali Italiano Dermatologia Venerologia 1987, 122, 599-604.
7. Walton et al, British Journal of Dermatology, 1986, 114, 261-264.
8. Cooper, Journal of Pharmaceutical Sciences, 1984.
9. Aungst B. J., Pharmaceutical Research 1989, 6, 244-247.
10. Sasaki H. et al. International Journal of Pharmaceutics 1990, 60, 177-183.
11. Mirejovsky D. and Takruri H., Journal of Pharmaceutical Sciences 1986, 75 1089-1093.
12. Chien, Transdermal controlled Systemic Medications, Marcel Dekker INC, New York 1987, pages 69-75

PATENTS

1. US Patent No. 3909816
2. US Patent No. 4311481
3. US Patent No. 4316893
4. US Patent No. 4537776 Cooper et al, August 27, 1985

## Claims

**1.** The use of an N,N-di($C_1$-$C_4$) alkyl lauramide as a permeation enhancer in combination with spironolactone for the manufacture of a medicament for topical administration to suppress increased androgenic activity of the skin and/or to treat skin conditions related to hyperandrogenic activity.

**2.** The use of an N,N-di($C_1$-$C_4$) alkyl lauramide as a permeation enhancer in the manufacture of a formulation for use in combination with spironolactone for topical administration to suppress increased androgenic activity of the skin and/or to treat skin conditions related to hyperandrogenic activity.

**3.** The use as claimed in claim 1 or 2, in combination with at least one additional enhancer selected from:
(i) fatty acids having 14 to 22 carbon atoms and one or more double bonds,
(ii) substituted di($C_1$-$C_4$) alkyl fatty acids having 8 to 14 carbon atoms; and
(iii) N-methyl pyrrolidone.

**4.** The use as claimed in any preceding claim, wherein said N,N-di($C_1$-$C_4$) alkyl lauramide is N,N-dimethyl lauramide.

**5.** The use as claimed in claim 4, of a permeation enhancer selected from (a) N,N-dimethyl lauramide and oleic acid, (b) N,N-dimethyl lauramide and N-methyl pyrrolidone, and (c) N,N-dimethyl lauramide and neodecanoic acid.

**6.** The use as claimed in any preceding claim, in the manufacture of a medicament containing 0.1 to 10 wt% spironolactone.

**7.** The use as claimed in any preceding claim, wherein the N,N-di($C_1$-$C_4$) alkyl lauramide is present in the medicament in a concentration of 0.1 to 10 wt%.

**8.** The use as claimed in claim 3, wherein the amount of said at least one additional enhancer is from 0.1 to 10 wt%.

**9.** A pharmaceutical composition for the topical application of spironolactone to reduce increased androgenic activity of the skin comprising:
(a) 0.1 to 10 wt% spironolactone,
(b) a permeation enhancer comprising N,N-dialkyl lauramide;
and
(c) a pharmaceutically acceptable vehicle.

**10.** A pharmaceutical composition as claimed in claim 9, which further comprises at least one additional permeation enhancer.

11. A pharmaceutical composition as claimed in claim 9 or 10, wherein said vehicle includes at least one buffer, at least one preservative, at least one flavoring additive and/or at least one excipient adapted to yield a cream having a cosmetic appearance.

12. A pharmaceutical composition as claimed in claim 10, wherein at least one said additional permeation enhancer is selected from oleic acid, N-methyl-pyrrolidone and neodecanoic acid, and wherein the amount of said at least one additional enhancer ranges from 2.5 to 10 wt%.

13. A pharmaceutical composition as claimed in any one of claims 9 to 12, wherein said N,N-di($C_1$-$C_4$) lauramide is N,N-dimethyl-lauramide.

**Patentansprüche**

1. Verwendung eines N,N-Di($C_1$-$C_4$)-alkyllauramids als Durchdringungsverstärker in Kombination mit Spironolacton zur Herstellung eines Arzneimittels zur topischen Verabreichung, um eine erhöhte androgene Aktivität der Haut zu unterdrücken und/oder um Hautzustände zu behandeln, die mit hyperandrogener Aktivität zusammenhängen.

2. Verwendung eines N,N-Di($C_1$-$C_4$)-alkyllauramids als Durchdringungsverstärker bei der Herstellung einer Formulierung zur Verwendung in Kombination mit Spironolacton zur topischen Verabreichung, um eine erhöhte androgene Aktivität der Haut zu unterdrücken und/oder um Hautzustände zu behandeln, die mit hyperandrogener Aktivität zusammenhängen.

3. Verwendung nach Anspruch 1 oder 2; in Kombination mit mindestens einem zusätzlichen Verstärker gewählt aus:
   (i) Fettsäuren mit 14 bis 22 Kohlenstoffatomen und einer oder mehreren Doppelbindungen,
   (ii) substituierten Di($C_1$-$C_4$)alkylfettsäuren mit 8 bis 14 Kohlenstoffatomen; und
   (iii) N-Methylpyrrolidon.

4. Verwendung nach einem der vorhergehenden Ansprüche, worin das N,N-Di($C_1$-$C_4$)alkyllauramid N,N-Dimethyllauramid ist.

5. Verwendung nach Anspruch 4 eines Durchdringungsverstärkers gewählt aus (a) N,N-Dimethyllauramid und Ölsäure, (b) N,N-Dimethyllauramid und N-Methylpyrrolidon und (c) N,N-Dimethyllauramid und Neodecansäure.

6. Verwendung nach einem der vorhergehenden Ansprüche bei der Herstellung eines Arzneimittels, enthaltend 0,1 bis 10 Gew.-% Spironolacton.

7. Verwendung nach einem der vorhergehenden Ansprüche, worin das N,N-Di($C_1$-$C_4$)alkyllauramid in dem Arzneimittel in einer Konzentration von 0,1 bis 10 Gew.-% vorhanden ist.

8. Verwendung nach Anspruch 3, worin die Menge des mindestens einen zusätzlichen Verstärkers 0,1 bis 10 Gew.-% beträgt.

9. Pharmazeutische Zusammensetzung zur topischen Verabreichung von Spironolacton zum Verringern erhöhter androgener Aktivität der Haut, umfassend
   (a) 0,1 bis 10 Gew.-% Spironolacton,
   (b) einen Durchdringungsverstärker umfassend N,N-Dialkyllauramid; und
   (c) ein pharmazeutisch annehmbares Vehikel.

10. Pharmazeutische Zusammensetzung nach Anspruch 9, welche weiterhin mindestens einen zusätzlichen Durchdringungsverstärker umfaßt.

11. Pharmazeutische Zusammensetzung nach Anspruch 9 oder 10, worin das Vehikel mindestens einen Puffer, mindestens ein Konservierungsmittel, mindestens einen Aromazusatz und/oder mindestens ein Excipiens enthält, das darauf eingestellt ist, eine Creme mit einem kosmetischen Aussehen zu ergeben.

12. Pharmazeutische Zusammensetzung nach Anspruch 10, worin mindestens ein solcher zusätzlicher Durchdringungsverstärker gewählt wird aus Ölsäure, N-Methylpyrrolidon und Neodecansäure, und worin die Menge des mindestens einen zusätzlichen Verstärkers in dem Bereich von 2,5 bis 10 Gew.-% liegt.

13. Pharmazeutische Zusammensetzung nach einem der Ansprüche 9 bis 12, worin das N,N-Di($C_1$-$C_4$)-lauramid N,N-Dimethyllauramid ist.

**Revendications**

1. Utilisation d'un N,N-dialkyl(en $C_1$-$C_4$)lauramide comme un agent d'augmentation de la pénétration en combinaison avec la spironolactone pour la fabrication d'un médicament pour administration topique pour supprimer une activité androgénique accrue de la peau et/ou pour traiter des états cutanés liés à une activité hyperandrogénique.

2. Utilisation d'un N,N-dialkyl(en $C_1$-$C_4$)lauramide comme un agent d'augmentation de la pénétration dans la fabrication d'une composition destinée à être utilisée en combinaison avec la spironolactone pour l'administration topique pour supprimer une activité androgénique accrue de la peau et/ou pour traiter des états cutanés liés à une activité hyperandrogénique.

3. Utilisation selon la revendication 1 ou 2, en combinaison avec au moins un agent supplémentaire d'augmentation choisi parmi:
   (i) des acides gras ayant 14 à 22 atomes de carbone et une ou plusieurs double liaisons,
   (ii) des acides gras substitués par deux groupes alkyle en $C_1$-$C_4$ ayant 8 à 14 atomes de carbone,
   (iii) la N-méthylpyrrolidone.

4. Utilisation selon l'une quelconque des revendications précédentes, dans laquelle ledit N,N-dialkyl(en $C_1$-$C_4$)lauramide est le N,N-diméthyllauramide.

5. Utilisation selon la revendication 4, d'un agent d'augmentation de la pénétration choisi parmi (a) le N,N-diméthyllauramide et l'acide oléique, (b)le N,N-diméthyllauramide et la N-méthylpyrrolidone, et (c) le N,N-diméthyllauramide et l'acide néodécanoïque.

6. Utilisation selon l'une quelconque des revendications précédentes, dans la fabrication d'un médicament contenant de 0,1 à 10% en poids de spironolactone.

7. Utilisation selon l'une quelconque des revendications précédentes, dans laquelle le N,N-dialkyl(en $C_1$-$C_4$)lauramide est présent dans le médicament en une concentration de 0,1 à 10% en poids.

8. Utilisation selon la revendication 3, dans laquelle la quantité dudit au moins un agent supplémentaire d'augmentation est de 0,1 à 10% en poids.

9. Composition pharmaceutique pour l'application topique de spironolactone pour réduire une activité androgénique accrue de la peau comprenant:
   (a) de 0,1 à 10% en poids de spironolactone,
   (b) un agent d'augmentation de la pénétration comprenant un N,N-dialkyllauramide; et
   (c) un véhicule pharmaceutiquement acceptable.

10. Composition pharmaceutique selon la revendication 9, qui comprend en outre au moins un agent supplémentaire d'augmentation de la pénétration.

11. Composition pharmaceutique selon la revendication 9 ou 10, dans laquelle ledit véhicule comprend au moins un tampon, au moins un conservateur, au moins un parfum et/ou au moins un excipient approprié pour l'obtention d'une crème ayant l'aspect d'un produit cosmétique.

12. Composition pharmaceutique selon la revendication 10, dans laquelle ledit au moins un agent supplémentaire d'augmentation de la pénétration est choisi parmi l'acide oléique, la N-méthylpyrrolidone et l'acide néodécanoïque, et dans laquelle la quantité dudit au moins un agent supplémentaire d'augmentation de la pénétration est de 2,5 à 10% en poids.

**13.** Composition pharmaceutique selon l'une quelconque des revendications 9 à 12, dans laquelle ledit N,N-dialkyl(en $C_1$-$C_4$)lauramide est le N,N-diméthyllauramide.